# EUROPEAN PATENT APPLICATION

(11) **EP 4 317 402 A1**
(43) Date of publication of application: **07.02.2024**
(21) Application number: 22774671.6
(22) Date of filing: 31.01.2022
(51) Int. Cl.: C12M 1/12, B01D 35/06, C12N 7/02

(54) **FILTRATION MEMBER, CONCENTRATION APPARATUS, AND CONCENTRATION METHOD**

(30) Priority: 26.03.2021 JP 2021054192
(71) Applicant: Yokogawa Electric Corporation, Tokyo 180-8750 (JP)
(72) Inventor: INOUE, Kentaro, Musashino-shi, Tokyo 180-8750 (JP); TAGUCHI, Tomoyuki, Musashino-shi, Tokyo 180-8750 (JP); KATAYAMA, Hiroyuki, Tokyo 113-8654 (JP); MATSUI, Yasuhiro, Musashino-shi, Tokyo 180-8750 (JP)
(74) Representative: Zimmermann, Tankred Klaus
(86) International application number: PCT/JP2022/003698
(87) International publication number: WO 2022/201869

(57) **Abstract**

A filtration member 110 including pores having a pore diameter through which microorganisms may pass and configured to have a positive charge or a negative charge upon application of a voltage.

## Description

### CROSS-REFERENCE TO RELATED APPLICATION

The present application claims priority to and the benefit of Japanese Patent Application No. 2021-054192 filed March 26, 2021, the entire contents of which are incorporated herein by reference.

### TECHNICAL FIELD

The present disclosure relates to a filtration member, a concentration apparatus, and a concentration method.

### BACKGROUND

Negatively charged membrane methods are known as conventional treatment methods for detecting viruses in water. For example, Patent Literature (PTL) 1 describes a method including preparing a purified virus solution from a sample solution, concentrating the purified virus solution by a negatively charged membrane method or the like, and detecting a virus by extracting nucleic acid. The sample solution may be from environmental water such as river water, lake water, seawater, rainwater, and the like, drinking water such as well water, tap water, bottled water, and the like, sewage water, wastewater, pool water, water used in industry such as agricultural water, industrial water, refrigerant water, and the like.

### CITATION LIST

### Patent Literature

### PTL 1: JP 2015-195756 A

### SUMMARY

### (Technical Problem)

In a negatively charged membrane method, the negatively charged membrane needs to be replaced each time filtration is performed to secure sample water filtration performance. However, the replacement of the negatively charged membrane needs to be done manually, which requires time and labor. Further, the cost of purchasing negatively charged membranes for replacement and the time taken from purchase to receipt are required.

It would be helpful to provide a filtration member, concentration apparatus, and a concentration method that improve the convenience of processing by the negatively charged membrane method.

### (Solution to Problem)

A filtration member according to at least one embodiment comprises pores having a pore diameter through which microorganisms may pass and configured to have a positive charge or a negative charge upon application of a voltage. Accordingly, when sample water is supplied to the filtration member while the filtration member is charged due to application of the voltage, a virus charged by the filtration member is captured by the filtration member. When the application of the voltage to the filtration member is stopped, the virus desorbs from the filtration member. When flushing water is supplied to the filtration member in this state, the virus flows out from the filtration member together with the flushing water and is collected. Therefore, viruses may be reliably flushed out and removed from the filtration member, and therefore the filtration member may be used repeatedly without replacement. Accordingly, convenience is increased by reducing the time and labor required to replace a filtration member, as well as reducing the cost of purchasing negatively charged membranes for replacement and the time taken from purchase to receipt.

According to an embodiment, the filtration member comprises a mesh structure or perforated structure made of metal that defines the pores. Accordingly, the filtration member is able to capture a virus while the filtration member is charged when the voltage is applied, and to desorb a virus from the filtration member when the application of the voltage to the filtration member is stopped.

According to an embodiment, the filtration member comprises a structure that has a mesh structure or a perforated structure that defines the pores, the structure being coated by an electrically conductive coating or metal film. Accordingly, the filtration member is able to capture a virus while the filtration member is charged when the voltage is applied, and to desorb a virus from the filtration member when the application of the voltage to the filtration member is stopped.

According to an embodiment, the filtration member comprises a bundle of metal pipes that define the pores. Accordingly, the filtration member is able to capture a virus while the filtration member is charged when the voltage is applied, and to desorb a virus from the filtration member when the application of the voltage to the filtration member is stopped.

A concentration apparatus according to at least one embodiment comprises: the filtration member described above; a power supply configured to apply the voltage to the filtration member; a sample water supply disposed upstream of the filtration member and configured to supply sample water; a flushing water supply disposed upstream of the filtration member in parallel with the sample water supply and configured to supply flushing water; a sample water supply pipe connected to an upstream pipe disposed upstream of the filtration member and configured to supply the sample water from the sample water supply to the filtration member; a flushing water supply pipe branched from and connected to the upstream pipe and configured to supply the flushing water to the filtration member; an outlet disposed downstream of the filtration member and configured to discharge fluid externally; and a collection container disposed downstream of the filtration member in parallel with the outlet and configured to collect fluid. Accordingly, when the sample water is supplied to the filtration member while the filtration member is charged due to application of the voltage, a virus charged by the filtration member is captured by the filtration member. When the application of the voltage to the filtration member is stopped, the virus desorbs from the filtration member. When the flushing water is supplied to the filtration member in this state, the virus flows out from the filtration member together with the flushing water and is collected. Therefore, viruses may be reliably flushed out and removed from the filtration member, and therefore the filtration member may be used repeatedly without replacement. Accordingly, convenience is increased by reducing the time and labor required to replace a filtration member, as well as reducing the cost of purchasing negatively charged membranes for replacement and the time taken from purchase to receipt.

A concentration method according to at least one embodiment is performed by a concentration apparatus comprising: a filtration member comprising pores having a pore diameter through which microorganisms may pass and configured to have a positive charge or a negative charge upon application of a voltage; a power supply configured to apply the voltage to the filtration member; a sample water supply disposed upstream of the filtration member and configured to supply sample water; a flushing water supply disposed upstream of the filtration member in parallel with the sample water supply and configured to supply flushing water; a sample water supply pipe connected to an upstream pipe disposed upstream of the filtration member and configured to supply the sample water from the sample water supply to the filtration member; a flushing water supply pipe branched from and connected to the upstream pipe and configured to supply the flushing water to the filtration member; an outlet disposed downstream of the filtration member and configured to discharge fluid externally; and a collection container disposed downstream of the filtration member in parallel with the outlet and configured to collect fluid, the concentration method comprising: filtering the sample water by supplying the sample water from the sample water supply to the filtration member while the voltage is applied to the filtration member by the power supply; and collecting a concentrate of microorganisms captured by the filtration member by supplying the flushing water from the flushing water supply to the filtration member while the application of the voltage from the power supply to the filtration member is stopped. Accordingly, when the sample water is supplied to the filtration member while the filtration member is charged due to application of the voltage, a virus charged by the filtration member is captured by the filtration member. When the application of the voltage to the filtration member is stopped, the virus desorbs from the filtration member. When the flushing water is supplied to the filtration member in this state, the virus flows out from the filtration member together with the flushing water and is collected. Therefore, viruses may be reliably flushed out and removed from the filtration member, and therefore the filtration member may be used repeatedly without replacement. Accordingly, convenience is increased by reducing the time and labor required to replace a filtration member, as well as reducing the cost of purchasing negatively charged membranes for replacement and the time taken from purchase to receipt.

### (Advantageous Effect)

According to the present disclosure, the filtration membrane, the concentration apparatus and the concentration method are provided that improve the convenience of processing by the negatively charged membrane method.

### BRIEF DESCRIPTION OF THE DRAWINGS

In the accompanying drawings:
FIG. 1 is a schematic diagram of a concentration apparatus according to a comparative example, which collects a concentrate of virus particles from sample water;
FIG. 2 is a schematic diagram for explanation of a processing step of a negatively charged membrane method executed via the equipment illustrated in FIG. 1;
FIG. 3 is a schematic diagram for explanation of a processing step of the negatively charged membrane method executed via the equipment illustrated in FIG. 1;
FIG. 4 is a schematic diagram for explanation of a processing step of the negatively charged membrane method executed via the equipment illustrated in FIG. 1;
FIG. 5 is a schematic diagram of a concentration apparatus according to an embodiment of the present disclosure;
FIG. 6 is a schematic diagram for explanation of a first step of processing of a negatively charged membrane method executed by the concentration apparatus illustrated in FIG. 5; and
FIG. 7 is a schematic diagram for explanation of a second step of processing of the negatively charged membrane method executed by the concentration apparatus illustrated in FIG. 5.

### DETAILED DESCRIPTION

An embodiment of the present disclosure is described below with reference to the drawings.

First, a comparative example is described of equipment that collects concentrates of microorganisms such as bacteria and viruses by a negatively charged membrane method. The negatively charged membrane method is an example of processing performed to collect a concentrate having increased virus concentration by concentrating viruses from sample water. Microorganisms such as bacteria and viruses have a negative charge in neutral to alkaline water. Sample water subjected to the processing of the negatively charged membrane method is neutral to alkaline, and therefore such microorganisms have a negative charge in the sample water.

As an example, the present disclosure describes collection of a virus concentrate, but similar equipment and methods may be applied to other particulates such as bacteria. Viruses may be any known virus. Viruses may include, for example, double-stranded deoxyribonucleic acid (DNA) viruses, single-stranded DNA viruses, double-stranded ribonucleic acid (RNA) viruses, single-stranded RNA viruses, single-stranded RNA reverse transcription viruses, and double-stranded DNA reverse transcription viruses.

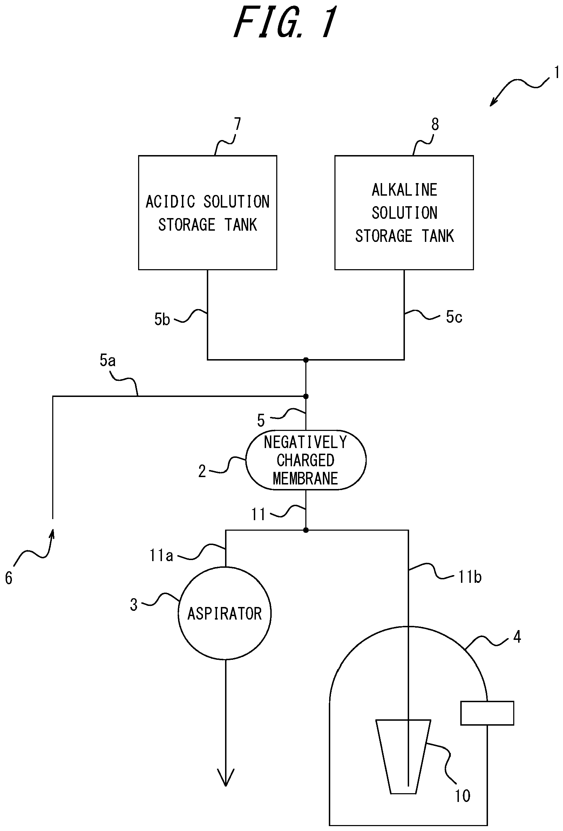

FIG. 1 is a schematic diagram for explanation of the comparative example of the equipment capable of performing processing by the negatively charged membrane method. As illustrated in FIG. 1, equipment 1 of the comparative example includes a negatively charged membrane 2, an aspirator 3, and a suction bottle 4. In FIG. 1, solid lines joining each component indicate piping through which fluid flows.

The negatively charged membrane 2 is a negatively charged membrane. For example, a mixed cellulose membrane manufactured by Millipore Corporation (hereinafter also referred to simply as "HA membrane") may be used. The mixed cellulose membrane is, for example, a mixed membrane of nitrocellulose and cellulose acetate. The negatively charged membrane 2 has pores through which viruses may pass and through which molecules that make up a fluid, such as water molecules, may permeate. The pore diameter of the pores in the negatively charged membrane 2 may be defined as appropriate.

A pipe 5 is provided upstream of the negatively charged membrane 2 to supply fluid. In the comparative example illustrated in FIG. 1, three different pipes 5a, 5b, and 5c are manually reconnected for each step, and different fluids are supplied to the negatively charged membrane 2 via the pipe 5. Instead of the pipes 5a, 5b, and 5c being reconnected to the pipe 5, different fluids may be supplied to the negatively charged membrane 2 via the pipe 5 by changing a solution fed into the pipe 5.

Of the three pipes 5a, 5b, and 5c, a first pipe 5a connects the pipe 5 to a sample water supply port 6. From the sample water supply port 6, sample water that may contain a virus is supplied to the first pipe 5a. The sample water supply port 6 may be supplied with a defined solution mixed with the sample water taken from a water treatment infrastructure facility. The defined solution may be, for example, a magnesium chloride solution. By supplying magnesium chloride solution, magnesium cations are adsorbed by a virus in the sample water, and a complex is formed of the virus and magnesium ions, which facilitates capture of the virus by the negatively charged membrane 2. As the defined solution, an appropriate solution may be used depending on properties of the sample water. Further, the defined solution need not be used, depending on the properties of the sample water.

Of the three pipes 5a, 5b, and 5c, a second pipe 5b connects the pipe 5 to an acidic solution storage tank 7 where an acidic aqueous solution is stored. The acidic aqueous solution is supplied to the second pipe 5b from the acidic solution storage tank 7. The acidic aqueous solution is described herein as a sulfuric acid solution as an example, but is not limited to this.

Of the three pipes 5a, 5b, and 5c, a third pipe 5c connects the pipe 5 to an alkaline solution storage tank 8 where an alkaline aqueous solution is stored. The alkaline aqueous solution is supplied to the third pipe 5c from the alkaline solution storage tank 8. The alkaline aqueous solution is described herein as a sodium hydroxide aqueous solution as an example, but is not limited to this.

Fluid is supplied to the negatively charged membrane 2 via the pipe 5 from each of the three pipes 5a, 5b, and 5c connected in a given step. Only one of the sample water, the acidic aqueous solution, and the alkaline aqueous solution is supplied to the negatively charged membrane 2 at a given time. In other words, the pipes 5a, 5b and 5c are manually reconnected for each step so that two or more of the sample water, the acidic aqueous solution, and the alkaline aqueous solution are not supplied at the same time.

The aspirator 3 is disposed downstream of the negatively charged membrane 2. The aspirator 3 draws in the fluid supplied to the negatively charged membrane 2 by creating a reduced pressure condition. In the comparative example illustrated in FIG. 1, in cases where the sample water and the acidic aqueous solution are supplied to the negatively charged membrane 2, the aspirator 3 is driven and fluid is drawn into the aspirator 3 and discharged externally.

The suction bottle 4 is disposed downstream of the negatively charged membrane 2 and in parallel with the aspirator 3. The suction bottle 4 draws in the fluid supplied to the negatively charged membrane 2 by creating a reduced pressure condition and collects the fluid in a concentrate collection container 10 provided internally. In the comparative example illustrated in FIG. 1, when the alkaline aqueous solution is supplied to the negatively charged membrane 2, the fluid is drawn into the suction bottle 4 and collected in the concentrate collection container 10.

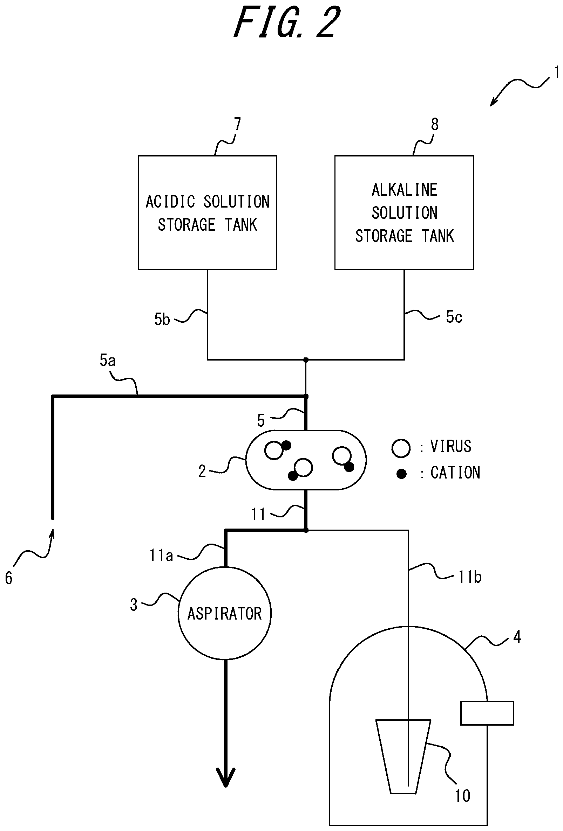

The following is a description of a processing method according to the negatively charged membrane method using the equipment of the comparative example illustrated in FIG. 1. FIG. 2 through FIG. 4 are schematic diagrams for explanation of processing steps of the negatively charged membrane method performed by the equipment 1 illustrated in FIG. 1. Bold lines in FIG. 2 through FIG. 4 indicate fluid flow. Here, a case is described where the sample water to be filtered is a small volume such as 2 liters or less. In this case, a flat membrane is used as the negatively charged membrane 2.

First, the first pipe 5a is connected to the pipe 5 and the aspirator 3 is driven to supply sample water from the sample water supply port 6 to the negatively charged membrane 2 via the first pipe 5a, as illustrated in FIG. 2. The sample water includes a complex of negatively charged viruses with cations adsorbed. As the sample water passes through the negatively charged membrane 2, the virus and cation complexes in the sample water are captured by adsorption on the negatively charged membrane 2 while passing through the negatively charged membrane 2. As the negatively charged membrane 2, one capable of allowing viruses to pass through at this time is used. For example, an HA membrane having a pore size of 0.45 µm and a diameter of 13 mm to 90 mm may be used as the negatively charged membrane 2. The sample water from which cations and viruses are captured is drained by the aspirator 3 through a pipe 11 and a pipe 11a disposed downstream of the negatively charged membrane 2.

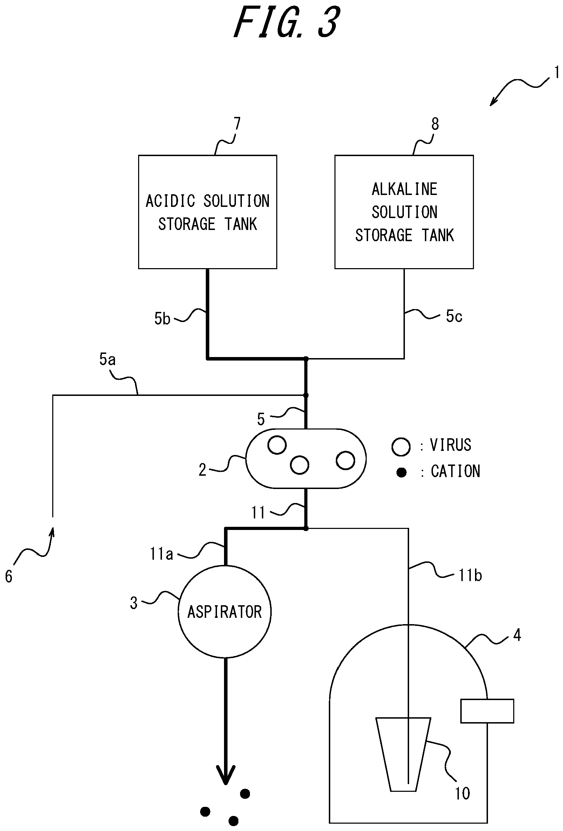

Next, the second pipe 5b is connected to the pipe 5 instead of the first pipe 5a, and the aspirator 3 is driven to supply the sulfuric acid solution from the acidic solution storage tank 7 through the second pipe 5b to the negatively charged membrane 2, as illustrated in FIG. 3. This results in acid washing of the negatively charged membrane 2. In other words, by supplying the sulfuric acid solution to the negatively charged membrane 2 and flowing downstream via the aspirator 3, the cations captured in the negatively charged membrane 2 are eluted from the negatively charged membrane 2 and drained with the sulfuric acid solution through the pipe 11 and the pipe 11a. Further, a surface of the virus captured by the negatively charged membrane 2 is positively charged due to the supplying of the sulfuric acid solution. The sulfuric acid solution may be any acid washing solution, for example 0.5 mM sulfuric acid solution at pH 3.0. The sulfuric acid solution may be supplied in an appropriate volume, for example, one-tenth or less of the volume of the sample water supplied. Acid washing leaves the negatively charged membrane 2 with viruses adhering thereto.

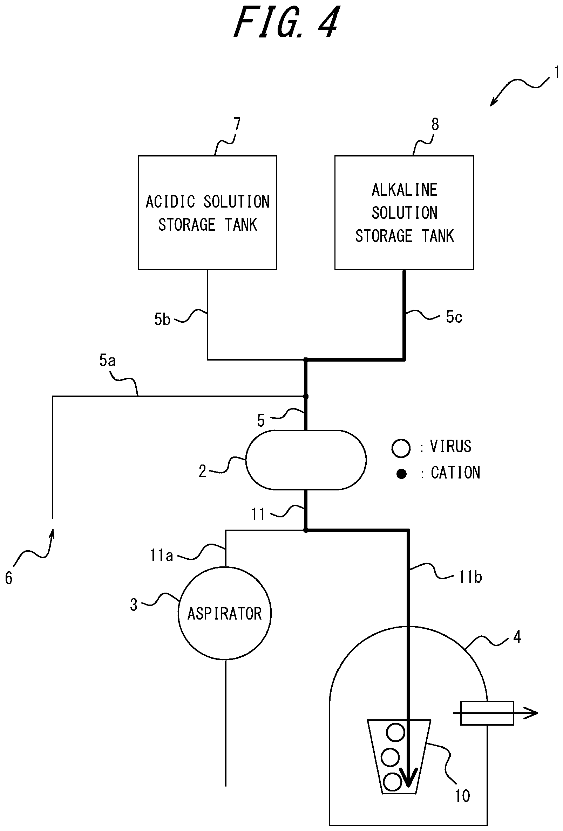

Then, the third pipe 5c is connected to the pipe 5 instead of the second pipe 5b, and sodium hydroxide aqueous solution is supplied to the negatively charged membrane 2 from the alkaline solution storage tank 8 through the third pipe 5c, as illustrated in FIG. 4. As a result, the surface of the virus captured by the negatively charged membrane 2 is again negatively charged, eluted from the negatively charged membrane 2, flows with the sodium hydroxide aqueous solution through the pipe 11 and the pipe 11b to the suction bottle 4, and is collected in the concentrate collection container 10. The sodium hydroxide aqueous solution may be any solution from which viruses may be collected, for example, a 1.0 mM sodium hydroxide aqueous solution at pH 10.5 to pH 10.8. The sodium hydroxide aqueous solution may be supplied in an appropriate volume, for example, 1 ml to 10 ml. The smaller the amount of sodium hydroxide aqueous solution supplied, the higher the effect of virus concentration, which is preferable.

The concentrate collection container 10 is preferably pre-filled with a solution to neutralize the sodium hydroxide aqueous solution from which the virus is collected. For example, the concentrate collection container 10 is preferably pre-filled with 5 µl to 50 µl of 0.2 N sulfuric acid solution and 10 µl to 100 µl of pH 8.0 buffer solution.

Thus, the processing described with reference to FIG. 2 through FIG. 4 may collect a virus in the sample water into a concentrate collection container 10 by performing the negatively charged membrane method using the equipment 1.

In the negatively charged membrane method described above, dispersion stability of a colloid is controlled by changing the surface charge of the virus according to pH dependent proton (for example, Mg²⁺ in the example above) addition and removal.

In the example described in PTL 1, particles (A) carry a positive charge as a surface charge. Therefore, viruses, humic acids, and particles (B) that carry a negative charge as a surface charge are adsorbed on particles (A). At this time, viruses are relatively less likely to adsorb than humic acids and particles (B), because viruses may carry a positive charge as a surface charge. As a result, viruses are not adsorbed on particles (A) and humic acids and particles (B) are adsorbed on particles (A). Particles (A) with humic acids or particles (B) adsorbed, that is, having a larger particle size, are then separated from a sample solution. Accordingly, humic acids and particles (B) adsorbed on particles (A) are also separated from the sample solution together with particles (A). In this way, a purified virus solution with humic acids removed is obtainable.

However, according to the conventional negatively charged membrane method, disposable negatively charged membranes 2 are used to prevent sample contamination and to secure filtration performance, and the negatively charged membrane 2 is replaced each time filtration is performed. However, the replacement of the negatively charged membrane 2 needs to be done manually, which requires time and labor. Further, the cost of purchasing the negatively charged membrane 2 for replacement and the time taken from purchase to receipt are required.

Further, when the negatively charged membrane 2 is configured as a flat membrane, the flat membrane is easily scratched. Therefore, when replacing the negatively charged membrane 2, care is required to avoid damaging the flat membrane. A cartridge filter may be used as the negatively charged membrane 2, but cartridge filters are expensive, which increases costs.

The following is a description of a filtration member, a concentration apparatus, and a concentration method that are able to solve the problems described above.

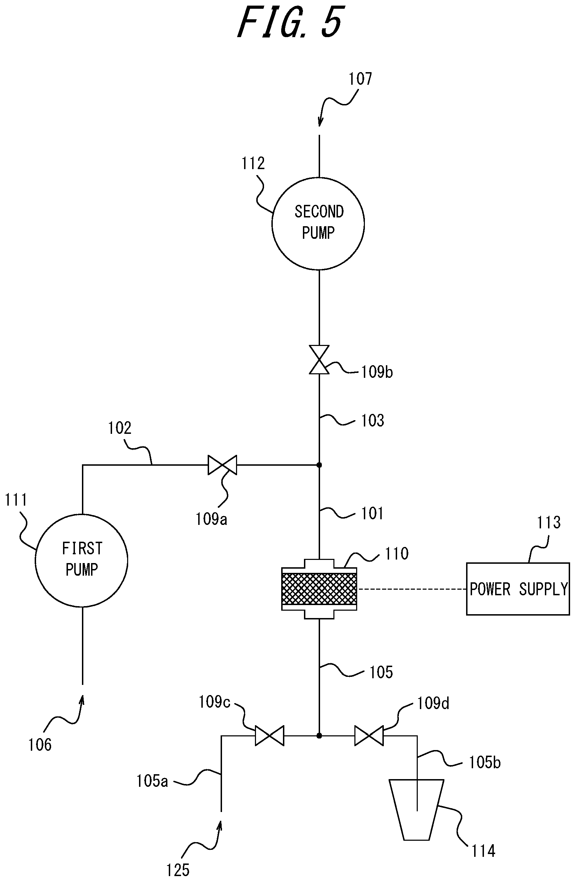

FIG. 5 is a schematic diagram of a concentration apparatus 100 according to an embodiment of the present disclosure. As illustrated in FIG. 5, the concentration apparatus 100 includes a filtration member 110, a first pump 111, a second pump 112, a power supply 113, and a concentrate collection container 114. In FIG. 5, solid lines joining each component indicate piping through which fluid flows. Further, in FIG. 5, the dashed line joining components indicates wiring able to supply electrical power.

The filtration member 110 is a member that filters fluid supplied from upstream. The filtration member 110 has pores of a pore diameter through which microorganisms, including viruses, are able to pass. For example, the filtration member 110 has a mesh structure or a perforated structure. In such a case, the filtration member 110 captures charged microorganisms and allows fluid such as water from which microorganisms have been separated to pass downstream via the pores formed by the mesh structure or the perforated structure. The pore diameter of the pores formed by the mesh structure or the perforated structure may be, for example, 0.1 µm to 30 µm. The perforated structure is a sheet or plate-like material having holes or a die-punched structure.

The filtration member 110 is electrically connected to the power supply 113, and configured to have voltage applied from the power supply 113. The filtration member 110 is composed of a member that may be charged to have a positive charge or a negative charge when a voltage is applied from the power supply 113. For example, the filtration member 110 is made of metal. In other words, the filtration member 110 may have a mesh structure or a perforated structure that is made of metal. For example, the filtration member 110 may be made of stainless steel that has controllable chargeability. Whether the filtration member 110 is charged to have a positive charge or a negative charge is controlled by the electrical power supplied by the power supply 113. The filtration member 110 has no charge when voltage is not applied from the power supply 113.

The filtration member 110 is preferably made of acid-resistant material. For example, the filtration member 110 is preferably made of acid-resistant material able to withstand pH 2 or lower. Accordingly, the filtration member 110 may be washed with a solution of strong acid after concentration by the concentration apparatus 100. When the filtration member 110 is made of acid-resistant material, then even when washed with a solution of strong acid, the filtration member 110 is less susceptible to corrosion by the solution of strong acid, and chargeability is not degraded.

According to the present embodiment, the filtration member 110 filters the sample water supplied from upstream in a charged state while a voltage is applied from the power supply 113.

The power supply 113 supplies electrical power to the filtration member 110. The power supply 113 supplies electrical power to the filtration member 110 when a switch is on and does not supply electrical power to the filtration member 110 when the switch is off. The power supply 113 may be switched on and off by a controller that controls the concentration apparatus 100 as a whole, for example, and may be switched on and off by a person operating the power supply 113, for example.

Upstream of the filtration member 110 is an upstream pipe 101 for supplying fluid. The upstream pipe 101 is connected to two pipes that branch upstream. In other words, two pipes, a sample water supply pipe 102 and a flushing water supply pipe 103, are connected to the upstream pipe 101. A sample water supply 106 is disposed upstream of the sample water supply pipe 102 to supply sample water. From the sample water supply 106, the sample water that may contain a virus is supplied to the sample water supply pipe 102. The sample water supplied from the sample water supply 106 to the sample water supply pipe 102 is supplied to the filtration member 110 via the upstream pipe 101. The sample water supply 106 may be supplied with a defined solution mixed with the sample water taken from a water treatment infrastructure facility, similar to that described for the comparative example. The defined solution is a solution able to supply cations. For example, the defined solution is a magnesium chloride solution or an aluminum chloride solution. However, the defined solution is not limited to magnesium chloride solution or aluminum chloride solution. By mixing in the magnesium chloride solution, a complex is formed by a virus and cations in the sample water, which makes the virus more easily captured by the filtration member 110 charged to have a negative charge. The sample water supply pipe 102 is provided with a first valve 109a. Opening and closing of the first valve 109a controls the supply of the sample water from the sample water supply pipe 102.

The sample water supply pipe 102 is provided with the first pump 111. The first pump 111 is provided upstream of the first valve 109a. The first pump 111 pumps the sample water downstream from the sample water supply 106. The first pump 111 may be a liquid feed pump or a pressure pump. For example, when filtering sample water that is a high load for the filtration member 110 due to high turbidity or insoluble substance concentration in the sample water, a pressure pump is preferably used as the first pump 111. By using a pressure pump, the sample water is supplied at higher pressure, and preventing precipitation of turbid or insoluble substances in the pipe is made easier.

A flushing water supply 107 that supplies flushing water is disposed upstream of the filtration member 110. The flushing water supply 107 may be configured, for example, as a mechanism to supply the flushing water via a hose, tube, or the like from a specific device able to supply the flushing water, and may be configured as a tank to store the flushing water. The flushing water supply 107 is disposed in parallel with the sample water supply 106. The flushing water is a fluid supplied to the filtration member 110 to flush out viruses captured by the filtration member 110 to flow downstream from the filtration member 110. The flushing water may be water, for example. However, other liquids may be used as the flushing water. The flushing water is supplied from the flushing water supply 107 to the filtration member 110 through the flushing water supply pipe 103, as illustrated in FIG. 5. Specifically, the flushing water supply pipe 103 is connected to the upstream pipe 101 to join the upstream pipe 101 downstream of the first valve 109a. In other words, the flushing water supply pipe 103 is branched from and connected to the upstream pipe 101. The flushing water supply pipe 103 is provided with a second valve 109b. Opening and closing of the second valve 109b controls the supply of the flushing water from the flushing water supply pipe 103.

The flushing water supply pipe 103 is provided with the second pump 112. The second pump 112 is provided upstream of the second valve 109b. The second pump 112 pumps the flushing water downstream from the flushing water supply 107. The second pump 112 may be a liquid feed pump.

Downstream of the filtration member 110 is a downstream pipe 105 for discharging fluid that has passed through the filtration member 110. Two different pipes, a downstream first pipe 105a and a downstream second pipe 105b, branch from and connect to the downstream pipe 105.

The downstream first pipe 105a is connected to an outlet 125 and discharges fluid externally from the outlet 125. The downstream first pipe 105a is provided with a third valve 109c. Opening and closing of the third valve 109c controls the discharge of fluid from the downstream first pipe 105a.

The downstream second pipe 105b connects the downstream pipe 105 to the concentrate collection container 114. The concentrate collection container 114 is discharged downstream of the filtration member 110, in parallel with the outlet 125. The concentrate collection container 114 is a container for collecting the virus concentrate. Specifically, the concentrate collection container 114 collects the flushing water including the virus due to the flushing out of the virus from the filtration member 110. The downstream second pipe 105b is provided with a fourth valve 109d. Opening and closing of the fourth valve 109d controls the pumping of concentrate from the second downstream pipe 105b to the concentrate collection container 114.

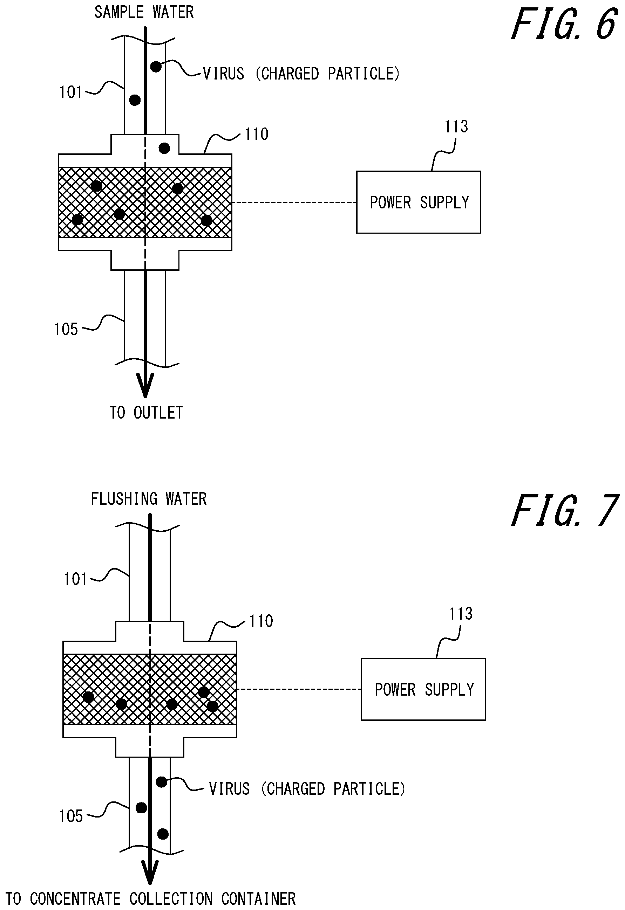

Next, processing steps of the negatively charged membrane method using the concentration apparatus 100 illustrated in FIG. 5 are described with reference to FIG. 6 and FIG. 7. FIG. 6 and FIG. 7 are schematic diagrams for explanation of steps of processing the negatively charged membrane method executed by the concentration apparatus 100. In FIG. 6 and FIG. 7, only the filtration member 110 and portions of the upstream pipe 101 and the downstream pipe 105 connected to the filtration member 110 are illustrated to make the main points easier to understand. In the concentration apparatus 100, the first step and the second step are performed to collect the concentrate of virus particles according to the negatively charged membrane method.

The first step and the second step may be performed automatically by a controller capable of automatically controlling each functional component of the concentration apparatus 100 according to a defined program. The first step and the second step may be performed by human operation. The following example assumes that the first step and the second step are automatically controlled.

FIG. 6 is a schematic diagram for explanation of the first step of the processing of the negatively charged membrane method executed by the concentration apparatus 100 illustrated in FIG. 5. In the first step, the sample water is filtered by supplying the sample water from the sample water supply 106 to the filtration member 110 while voltage is applied to the filtration member 110 by the power supply 113.

Specifically, in the first step, the first valve 109a and the third valve 109c are open, and the second valve 109b and the fourth valve 109d are closed. Further, the power supply 113 is switched on and electrical power is supplied to the filtration member 110, which electrically charges the filtration member 110. The following example assumes that the filtration member 110 has a positive charge when electrically charged.

In this state, the first pump 111 is driven to supply the sample water, which may contain a virus, from the sample water supply 106 to the filtration member 110 via the sample water supply pipe 102. The sample water supplied to the filtration member 110 is filtered by the filtration member 110. At this time, a virus in the sample water is negatively charged and the filtration member 110 is positively charged, and therefore the negatively charged virus is captured by the filtration member 110, as schematically illustrated in FIG. 6. The sample water from which the virus is removed is discharged from the downstream pipe 105, through the downstream first pipe 105a, and out of the outlet 125.

The voltage applied to the filtration member 110 while the sample water is being supplied to the filtration member 110, as illustrated in FIG. 6, may be determined according to the properties of the virus to be captured, the properties of the sample water, and the like. For example, the voltage applied to the filtration member 110 may be set from 1 mV to 1.23 V to prevent electrolysis of the sample water from occurring. However, when the effect of electrolysis is considered small, based on the flow velocity of the sample water and the speed of electrolysis, the voltage applied to the filtration member 110 may be set higher than 1.23 V.

FIG. 7 is a schematic diagram for explanation of the second step of the processing of the negatively charged membrane method executed by the concentration apparatus 100 illustrated in FIG. 5. In the second step, with the application of the voltage from the power supply 113 to the filtration member 110 stopped, the flushing water is supplied to the filtration member 110 from the flushing water supply 107 to collect a concentrate of microorganisms captured by the filtration member 110.

Specifically, in the second step, the first valve 109a and the third valve 109c are closed, and the second valve 109b and the fourth valve 109d are open. Further, the power supply 113 is switched off and electrical power is not supplied to the filtration member 110. In other words, the filtration member 110 is in an uncharged state.

In this state, the second pump 112 is driven to supply the flushing water from the flushing water supply 107 to the filtration member 110 via the flushing water supply pipe 103. At this time, the virus desorbs from the mesh structure or the perforated structure of the filtration member 110 because the filtration member 110 is not electrically charged. Therefore, as schematically illustrated in FIG. 7, the flushing water flushes viruses out of the filtration member 110 and downstream. The flushing water including the virus flows from the downstream second pipe 105b to the concentrate collection container 114 and is collected in the concentrate collection container 114.

When concentration is complete, the filtration member 110 may be washed and used again. In other words, contamination problems are unlikely to occur in the concentration apparatus 100 even when the filtration member 110 is reused without being replaced. This is because, in the second step of the concentration method according to the present embodiment, the virus desorbs from the filtration member 110 due to the stopping of the application of the voltage to the filtration member 110 and is flushed from the filtration member 110 by the flushing water, and therefore the virus is unlikely to remain in the filtration member 110.

When the filtration member 110 is made of acid-resistant material, the filtration member 110 may be washed with a solution of strong acid after concentration is complete. By washing the filtration member 110 with a solution of strong acid, when the virus remains in the filtration member 110, nucleic acids such as ribonucleic acid (RNA) and deoxyribonucleic acid (DNA) of the virus may be dissolved. Accordingly, the filtration member 110 is washed, and therefore the same filtration member 110 may be used for subsequent processing without contamination or other effects on a sample used in the subsequent processing.

Thus, the concentration apparatus 100 includes the filtration member 110 that is electrically charged when a voltage is applied. In a state where the voltage is applied and the filtration member 110 is electrically charged, the virus charged by the filtration member 110 is captured by the filtration member 110 when the sample water is supplied to the filtration member 110. The captured virus desorbs from the filtration member 110 when the application of the voltage to the filtration member 110 is stopped, and when the flushing water is supplied to the filtration member 110 in this state, the virus flows out of the filtration member 110 with the flushing water to be collected. This concentration method reliably flushes out and removes viruses from the filtration member 110, and therefore the filtration member 110 may be used repeatedly without replacement. Therefore, convenience is increased by reducing the time and labor required to replace the filtration member 110, as well as reducing the cost of purchasing negatively charged membranes for replacement and the time taken from purchase to receipt.

In the concentration apparatus 100 according to the embodiment described above, when the flushing water is water, a target virus may be collected without imparting stress from an acid, alkali, or the like. In other words, damage done to the virus may be reduced.

However, acid washing and alkaline flushing may also be performed in the second step of the concentration method according to the embodiment described above. In other words, instead of the flushing water, a virus captured by the filtration member 110 may be collected by supplying an aqueous solution of an acid such as a sulfuric acid solution and the like, and an aqueous solution of an alkali such as a sodium hydroxide aqueous solution and the like to the filtration member 110, as explained in reference to the comparative example.

According to the embodiment above, the sample water and the flushing water are supplied downstream by being pumped by the first pump 111 and the second pump 112, respectively, which are disposed upstream of the filtration member 110. However, instead of the first pump 111 and the second pump 112, the concentration apparatus 100 may include a pump downstream of the filtration member 110 that draws fluid (that is, the sample water and the flushing water) from upstream to downstream.

According to the embodiment above, the filtration member 110 has the mesh structure or the perforated structure that is made of metal. However, the filtration member 110 does not necessarily have to have the mesh structure or the perforated structure that is made of metal. It suffices that the filtration member 110 is chargeable by the application of a voltage and capable of filtering the sample water.

For example, the filtration member 110 may be formed by coating an electrically conductive paint or metal film on a structure that has a mesh structure or a perforated structure in which pores are formed. In such a case, the filtration member 110 functions in the same manner as the filtration member 110 described according to the embodiment above.

Further, for example, the filtration member 110 may be formed by bundling metal pipes that form pores. The metal pipes are arranged so that hollow portions forming the pores extend along the direction in which the sample water flows. In such a case, when the voltage is applied to the filtration member 110, the metal pipes forming the filtration member 110 are charged and a virus is captured (adsorbed) on the inner walls of the metal pipes. When the filtration member 110 is formed by bundling the metal pipes, the closer the distance between a target virus and the inner wall surfaces constituting the pores of the metal pipes, the more efficient the capture of the virus by the metal pipes. Therefore, metal pipes having smaller inner diameters are preferred. The inner diameter of each of the metal pipes may be 20 µm, for example.

In the concentration apparatus 100 according to the embodiment described above, the voltage applied to the filtration member 110 may be determined according to the microorganism to be concentrated. For example, the surface charge of various viruses, including noroviruses, Escherichia coli phage viruses, and the like, varies depending on the pH of the liquid including the virus. Therefore, the charge of the virus adsorption mechanism may be switched to a positive charge or a negative charge by changes in the pH or salt concentration of the liquid. This property can be used to collect a specific virus of interest by providing the filtration member 110 with an electrical charge that attracts the charged virus.

Specifically, a specific virus may be selectively collected by switching or adjusting the positive or negative voltage applied to the filtration member 110, or by switching whether or not the voltage is applied. For example, under pH 7 conditions, outer shell proteins of intestinal Picornaviridae and Tymoviridae that infect plants have a property of becoming positively charged, while Caliciviridae that cause gastroenteritis and the like have a property of becoming negatively charged. When the sample water contains a mixture of these viruses, then a norovirus belonging to the family Caliciviridae, for example, may be selectively captured by positively charging the filtration member 110. In this way, a concentrate of a target virus may be collected that does not contain a non-target virus.

The above embodiment describes an example in which the concentration apparatus 100 collects a concentrate of microorganisms such as bacteria and viruses, but the concentration apparatus 100 is equally applicable to particulate and colloidal dispersions that are charged and in a liquid.

The concentration apparatus 100 described above may be used in a variety of fields and applications. For example, the concentration apparatus 100 described above may be used to monitor water quality management and treatment performance of water treatment infrastructure such as water purification plants, sewage treatment plants, water reclamation facilities, and seawater desalination facilities. The concentration apparatus 100 described above may also be used, for example, to conduct dynamic surveys of the environment in water bodies such as rivers, oceans, water features, swimming pools, bathing areas, and the like. The concentration apparatus 100 described above may also be used, for example, for water quality testing for particulate and colloidal dispersion, microorganisms, and the like to determine the risk of microbial infection in a city including water bodies and environmental infrastructure. The concentration apparatus 100 described above may also be used for the purpose of qualitative risk, safety monitoring, or quality control of liquids used for beverages or in the production of processed foods, to quantify risk or to verify a comparison with a threshold value to determine safety. The concentration apparatus 100 described above may also be used for testing the quality of water for industrial, irrigation, and agricultural use. The concentration apparatus 100 described above may be used, for example, to manage qualitative risk, safety monitoring, or quality control of liquids used for temperature and humidity management, such as in mist spraying, humidification devices, or water sprinkling. The concentration apparatus 100 described above may also be used for quality control testing of water related to medical care such as water used in pharmaceutical manufacturing, dialysis therapy, and the like.

Further, the concentration apparatus 100 may selectively collect viruses as described above, and therefore may be utilized, for example, in the fields of water supply, food, drinking water, and other fields where particles having different charge states are targeted for collection. Further, by not using acidic or alkaline solutions as the flushing water, a virus is not stressed by acid, alkali, or the like, and therefore culturing after virus collection may be possible.

The present disclosure is not limited to the configurations specified in the embodiments described above, and various modifications are possible without departing from the scope of the claims. For example, functions included in each component, each step, and the like may be reconfigured and multiple components or steps may be combined into one or divided, as long as no logical inconsistency results.

### REFERENCE SIGNS LIST

- 1: Equipment
- 2: Negatively charged membrane
- 3: Aspirator
- 4: Suction bottle
- 5, 11, 11a, 11b: Pipe
- 5a: First pipe
- 5b: Second pipe
- 5c: Third pipe
- 6: Sample water supply port
- 7: Acidic solution storage tank
- 8: Alkaline solution storage tank
- 10: Concentrate collection container
- 100: Concentration apparatus
- 101: Upstream pipe
- 102: Sample water supply pipe
- 103: Flushing water supply pipe
- 105: Downstream pipe
- 105a: Downstream first pipe
- 105b: Downstream second pipe
- 106: Sample water supply
- 107: Flushing water supply
- 109a: First valve
- 109b: Second valve
- 109c: Third valve
- 109d: Fourth valve
- 110: Filtration member
- 111: First pump
- 112: Second pump
- 113: Power supply
- 114: Concentrate collection container
- 125: Outlet

## Claims

1. A filtration member comprising pores having a pore diameter through which microorganisms may pass and configured to have a positive charge or a negative charge upon application of a voltage.

2. The filtration member according to claim 1, comprising a mesh structure or perforated structure made of metal that defines the pores.

3. The filtration member according to claim 1, comprising a structure that has a mesh structure or a perforated structure that defines the pores, the structure being coated by an electrically conductive coating or metal film.

4. The filtration member according to claim 1, comprising a bundle of metal pipes that define the pores.

5. A concentration apparatus comprising:
the filtration member according to any one of claims 1 to 4;
a power supply configured to apply the voltage to the filtration member;
a sample water supply disposed upstream of the filtration member and configured to supply sample water;
a flushing water supply disposed upstream of the filtration member in parallel with the sample water supply and configured to supply flushing water;
a sample water supply pipe connected to an upstream pipe disposed upstream of the filtration member and configured to supply the sample water from the sample water supply to the filtration member;
a flushing water supply pipe branched from and connected to the upstream pipe and configured to supply the flushing water to the filtration member;
an outlet disposed downstream of the filtration member and configured to discharge fluid externally; and
a collection container disposed downstream of the filtration member in parallel with the outlet and configured to collect fluid.

6. A concentration method performed by a concentration apparatus comprising:
a filtration member comprising pores having a pore diameter through which microorganisms may pass and configured to have a positive charge or a negative charge upon application of a voltage;
a power supply configured to apply the voltage to the filtration member;
a sample water supply disposed upstream of the filtration member and configured to supply sample water;
a flushing water supply disposed upstream of the filtration member in parallel with the sample water supply and configured to supply flushing water;
a sample water supply pipe connected to an upstream pipe disposed upstream of the filtration member and configured to supply the sample water from the sample water supply to the filtration member;
a flushing water supply pipe branched from and connected to the upstream pipe and configured to supply the flushing water to the filtration member;
an outlet disposed downstream of the filtration member and configured to discharge fluid externally; and
a collection container disposed downstream of the filtration member in parallel with the outlet and configured to collect fluid,
the concentration method comprising:
filtering the sample water by supplying the sample water from the sample water supply to the filtration member while the voltage is applied to the filtration member by the power supply; and
collecting a concentrate of microorganisms captured by the filtration member by supplying the flushing water from the flushing water supply to the filtration member while the application of the voltage from the power supply to the filtration member is stopped.
